(19) Europäisches Patentamt
European Patent Office
Office européen des brevets

(11) Veröffentlichungsnummer: **0 427 942 A2**

(12) **EUROPÄISCHE PATENTANMELDUNG**

(21) Anmeldenummer: **90118765.8**

(51) Int. Cl.[5]: **C07D 403/12**, C08K 5/43

(22) Anmeldetag: **29.09.90**

(30) Priorität: **16.11.89 DE 3938015**

(43) Veröffentlichungstag der Anmeldung:
**22.05.91 Patentblatt 91/21**

(84) Benannte Vertragsstaaten:
**AT BE CH DE DK ES FR GB GR IT LI LU NL SE**

(71) Anmelder: **Degussa Aktiengesellschaft**
**Weissfrauenstrasse 9**
**W-6000 Frankfurt am Main 1(DE)**

(72) Erfinder: **Krimmer, Hans-Peter, Dr.**
**Am Weissen Turm 48**
**W-6000 Frankfurt/M.-60(DE)**
Erfinder: **Drauz, Karlheinz, Dr.**
**Zur Marienruhe 13**
**W-6463 Freigericht 1(DE)**
Erfinder: **Wolff, Siegfried, Dr.**
**Weiherstrasse 28**
**W-5303 Bornheim-Merten(DE)**
Erfinder: **Görl, Udo, Dr.**
**Quittenstrasse 36**
**W-5309 Meckenheim(DE)**

(54) **Verfahren zur Herstellung von s-Triazinsulfenimiden und deren Verwendung.**

(57) Die Erfindung betrifft ein Verfahren zur Herstellung von s-Triazinsulfenamiden der allgemeinen Formel

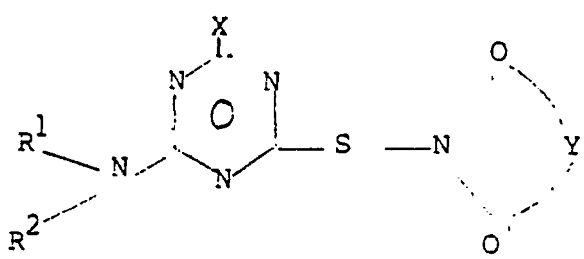

(I),

bei dem man ein s-Triazinsulfenamid der allgemeinen Formel

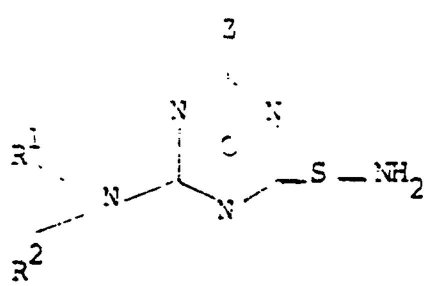

(II)

mit einem cyclischen Dicarbonsäureanhydrid der allgemeinen Formel

EP 0 427 942 A2

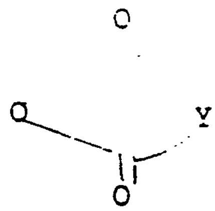

(III)

umsetzt. Die s-Triazinsulfenamide werden in vulkanisierbaren Kautschukmischungen als Verzögerer und Beschleuniger einsetzt.

# HERSTELLUNG VON S-TRIAZINSULFENIMIDEN UND DEREN VERWENDUNG

Die Erfindung betrifft ein neues Verfahren zur Herstellung von Triazinsulfenimiden, die am symetrischen oder 1,3,5-Triazinring ein- oder zweimal eine Sulfenimidgruppe, die sich von Dicarbonsäuren ableitet, aufweisen, und ihre Verwendung in vulkanisierbaren Kautschukmischungen.

Triazinsulfenimide von Dicarbonsäuren sind bekannt. In der DE-PS 24 30 143 wird ihre Darstellung durch Umsetzung von Triazinsulfenylchloriden mit cyclischen Dicarbonsäureimiden beschrieben.

Das Verfahren besitzt jedoch den Nachteil, daß als Zwischenprodukte die instabilen und hydrolyseempfindlichen Sulfenylchloride aus den Mercaptoverbindungen hergestellt werden müssen. Dies geschieht durch Chlorierung mit elementarem Chlor oder mit Sulfurylchlorid. In beiden Fällen entsteht als Nebenprodukt Chlorwasserstoff, der das Triazinmolekül protoniert.

Dieser muß von der Abtrennung der Endprodukte mit zusätzlichen Mengen einer organischen Base neutralisiert werden, so daß eine große Menge des Hydrochlorids der Base als Nebenprodukt anfällt.

Bei der Verwendung von Sulfurylchlorid als Chlorierungsmittel entstehen zusätzlich molare Mengen an Schwefeldioxid als Nebenprodukt, die umständlich über eine Absorberkolonne aufgefangen werden müssen.

Die Sulfenylchloride sind thermisch labil und können nicht über größere Zeiträume gelagert werden. Zudem bilden sich bei der Darstellung höhermolekulare Nebenprodukte, die die Ausbeute reduzieren.

Ein weiterer Nachteil des bekannten Verfahrens besteht darin, daß als Reaktionspartner der Sulfenylchloride cyclische Dicarbonsäureimide verwendet werden müssen. Diese werden technisch aus den entsprechenden Anhydriden dargestellt und sind deshalb teurere Ausgangsprodukte als die Anhydride.

Aufgabe der Erfindung ist es, ein Verfahren bereitzustellen, das unter Verwendung leichter verfügbarer Verbindungen und auf einem leichteren Weg zu den gewünschten Sulfenimiden führt.

Gegenstand der Erfindung ist ein Verfahren zur Herstellung von s-Triazinsulfenimiden der allgemeinen Formel

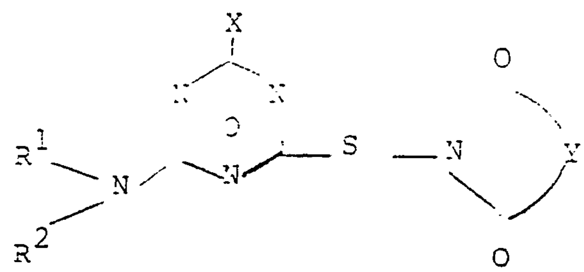

, (I)

in der bedeuten:

X:

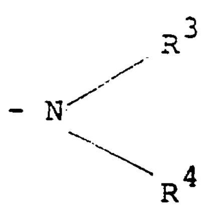

oder

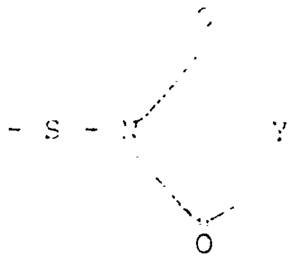

$R^1$, $R^2$, $R^3$ und $R^4$, gleich oder verschieden: Wasserstoff, Alkyl mit 1 bis 4 Kohlenstoffatomen, Alkenyl mit 3 bis 5 Kohlenstoffatomen, Cycloalkyl mit 3 bis 8 Kohlenstoffatomen, letzteres insbesondere unter der Voraussetzung, daß $R^1$ und$R^3$ Wasserstoff bedeuten, wenn die benachbarten Substituenten $R^2$ bzw. $R^4$ Cycloalkyl entsprechen.

$R^1$ mit $R^2$ und/oder $R^3$ mit $R^4$ können ringförmig miteinander verknüpft sein, so gestalt,

daß 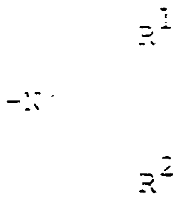 und oder 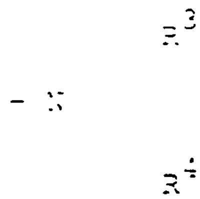 gemeinsam

einen Pyrrolidino-, Piperidino-, Morpholino-, 3,5-Dimethylmopholino- oder 4-N-Alkylpiperazinoring bilden;

Y: die Gruppierungen

$- CH_2 - CH_2 -$

$- CH_2 - CH_2 - CH_2 -$

$- CH(CH_3) - CH_2 -$

$- CH = CH -$

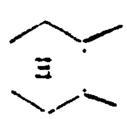
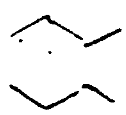
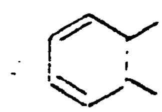

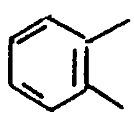
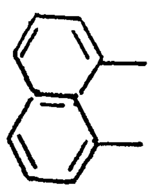
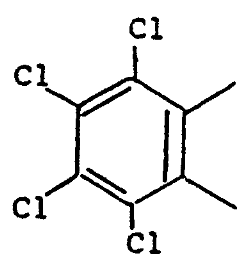

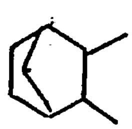
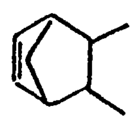
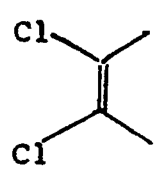

dadurch gekennzeichnet, daß man ein s-Triazinsulfenamid der Formel

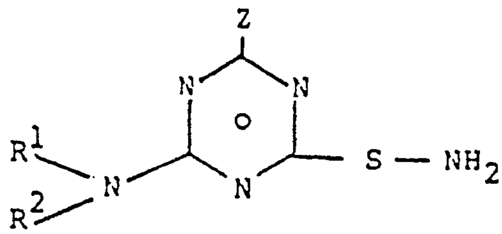

in der bedeuten
Z:

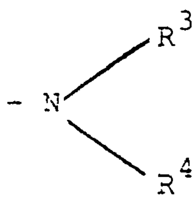

oder
- S - $NH_2$
und $R^1$, $R^2$, $R^3$ und $R^4$ die oben aufgeführte Bedeutung haben, mit einem cyclischen Dicarbonsäureanhydrid der Formel

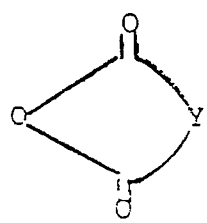

(III),

in der Y die oben aufgeführte Bedeutung besitzt, in einem inerten, wasserunlöslichen organischen Lösungsmittel bei Temperaturen zwischen 20 ° und 150 ° C, insbesondere 40 und 100 °C, vorzugsweise der Siedetemperatur des verwendeten Lösungsmittels umsetzt.

Sulfenamide der allgemeine Formel (II) sind stabile, kristalline Substanzen, die in hohen Ausbeuten aus den entsprechenden Mercaptoverbindungen gemäß DE-PS 20 27 432 hergestellt werden können.

Die Umsetzung zu den gewünschten Triazinsulfenimiden der allgemeinen Formel (I) erfolgt am besten in einem Lösungsmittel, das beim Sieden Wasser azeotrop mitschleppt. Das durch die Kondensation gebildete Reaktionswasser kann an einem aufgesetzten Wasserrabscheider aufgefangen, und die Reaktion aufgrund der erhaltenen Menge Wasser kontrolliert werden.

Die beiden Reaktionspartner, das Sulfenamid gemäß Formel (II) und das Anhydrid gemäß Formel (III) werden in äquimolaren Mengen eingesetzt, wenn Z für die Aminogruppe steht.
Hat Z die Bedeutung einer Sulfenamidogruppe, wählt man 1 : 2 als molares Verhältnis von Triazin-Verbindung und Anhydrid.

Als Lösungsmittel besonders geeignet ist ein wasserschleppendes Solvens, wie z. B. ein chlorierter Wasserstoff, wie Dichlormethan, Chloroform, Tetrachlorkohlenstoff, 1,2-Dichlorethan oder ein aliphatischer oder aromatischer Kohlenwasserstoff, wie Cyclohexan, Benzol, Toluol oder Xylol.

Die bevorzugte Reaktionstemperatur richtet sich jeweils nach dem Siedepunkt des verwendeten Lösungsmittel.

Die Reihenfolge, in der die beiden Reaktionspartner im Lösungsmittel gelöst werden, unterliegt keiner Beschränkung.
Nach ca. 4 bis 12 Stunden ist die Reaktion beendet.

Durch die Zugabe einer katalytischen Menge (~0,01 bis 1 Gew.-%, bezogen auf die Gesamtmenge der Reaktionsmischung) einer starken anorganischen Säure, wie z. B. konz. Schwefelsäure oder Phosphorsäure, oder einer organischen Säure, wie z. B. p-Toluolsulfonsäure, oder einem stark sauren Ionenaustauscher

kann die Reaktionszeit um etwa die Hälfte verkürzt werden.

Nach Beendigung der Reaktion scheidet sich das Produkt entweder bei Abkühlen ab, oder man destilliert das Lösungsmittel vorzugsweise unter Anwendung von Vakuum, ab. Dabei fällt das gebildete Reaktionsprodukt zumeist bereits beim Einengen aus. Ansonsten kann man das Produkt durch Versetzen des Rückstandes mit einem aliphatischen Kohlenwasserstoff, wie z. B. Petrolether, Hexan oder Cyclohexan, ausfällen.

Die Erfindung betrifft weiterhin die Verwendung von s-Triazinsulfenimiden in Kombination mit N,N′-substituierten Bis-(2,4-diamino-s-triazin-6-yl)tetra- oder polysulfiden in vulkanisierbaren Kautschukmischungen als Vulkanisationsverzögerer mit einem die Vernetzungsdichte erhöhendem Effekt und als Vulkanisationsbeschleuniger.

Bekanntlich werden Kautschukmischungen nach der Herstellung in entsprechenden Mischgeräten noch vor der Vulkanisation einem Formgebungsprozeß unterworfen. Dieser Formgebungsprozeß ist in jedem Fall mit wärmeerzeugenden mechanischen Beanspruchungen wie Walzen, Spritzen oder Kalandrieren verbunden und findet bei erhöhten Temperaturen statt. Die Grenze der Verarbeitungssicherheit ist dann erreicht bzw. überschritten, wenn dabei schon die Vulkanisation einsetzt. Dann ist nämlich eine maßhaltige plastische Formgebung nicht mehr möglich. Diese Grenze kann durch Einsatz der erfindungsgemäßen Substanzen zu höheren Temperaturen oder längeren Verarbeitungszeiten hin verschoben werden.

Die erfindungsgemäß hergestellten Triazinsulfenimide sind in Kautschukmischungen wirksam, die mit Schwefel vulkanisierbare(n) natürlichen und/oder synthetische(n) Kautschuk(e) enthalten.

Die Verwendung erfolgt in an sich bekannten Kautschukmischungen, die als weitere übliche Mischungskomponenten enthalten können:

- Verstärkungssystem, d. h. Furnace-Ruße, Channel-Ruße, Flammruße, Thermalruße, Acetylenruße, Lichtbogenruße, CK-Ruße usw. sowie synthetische Füllstoffe, wie Kieselsäuren, Silikate, Aluminiumoxidhydrate, Calciumcarbonate und natürliche Füllstoffe wie Clays, Kieselkreiden, Kreiden, Talke usw. sowie silanmodifizierte Füllstoffe und deren Verschnitte in Mengen von 5 bis 300 Teilen je 100 Teile Kautschuk
- Zinkoxid und Stearinsäure als Promotoren der Vulkanisation in Mengen von 0,5 bis 10 Teilen pro 100 Teile Kautschuk,
-üblicherweise verwendete Alterungs-, Ozon-, Ermüdungsschutzmittel wie z. B. IPPD,TMQ usw. und auch Wachse als Lichtschutzmittel und deren Verschnitte,
- beliebige Weichmacher wie z. B. aromatischem, naphthenische, paraffinische, synthetische Weichmacher und deren Verschnitte,
- gegebenenfalls in der Kautschukindustrie dem Stand der Technik entsprechend verwendete Organosilane, wie z. B. $\gamma$-Chlorpropyltrialkoxysilane, Vinyltrialkoxysilane, $\gamma$-Mercaptopropyltrialkoxysilane und Aminoalkyltrialkoxysilane sowie deren Verschnitte in einer Menge von 0,1 bis 25, bevorzugt 1 bis 10 Teile, je 100 Teile silanolgruppentragender Füllstoffe wie Kieselsäuren, Silikate, Clays usw.,
- gegebenenfalls Farbstoffe und Verarbeitungshilfsmittel in der üblichen Dosierung.

Zu den synthetischen Kautschuken zählen z. B. Polybutadiene, Polyisoprene, Butadien-Styrol-Copolymere, Butadien-Acrylnitril-Copolymere,Butylkautschuk, Terpolymere aus Ethylen, Propylen und einem nicht konjugierten Dien, das Trans-Polypentenamer und andere bekannte in der Kautschukindustrie verarbeitete Elastomere.

Die erfindungsgemäßen s-Triazinderivate sind in den Kautschukmischungen in Mengen von 0,1 bis 10 Gewichtsteilen auf 100 Gewichtsteile Kautschuk, insbesondere in Mengen von 0,1 bis 5 Gewichtsteilen, vorzugsweise 0,3 bis 3 Gewichtsteilen, jeweils bezogen auf 100 Gewichtsteile Kautschuk, enthalten.

Als Beschleuniger für die Vulkanisation werden erfindungsgemäß oligosulfidische Triazinverbindungen gemäß Formel (IV) eingesetzt, wie sie in den deutschen Patentanmeldungen P 34 38 890 und 36 10 794 beschrieben sind.

Dabei handelt es sich um folgenden Verbindungen:

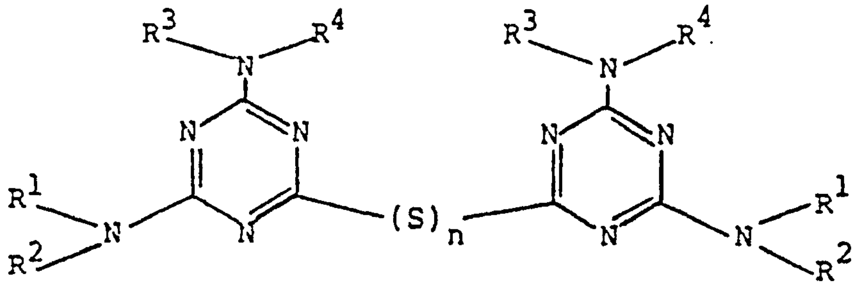

(IV)

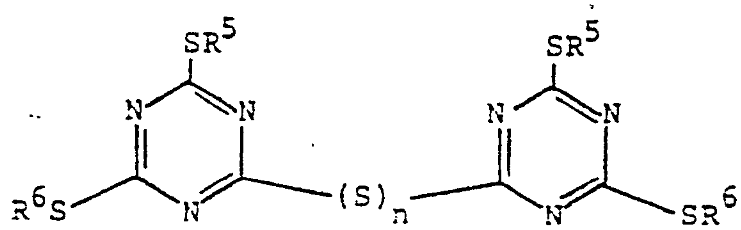

in welcher bedeuten:
$R^1$, $R^2$ = H;
$R^2$, $R^3$, $R^4$ = H, $C_1$-$C_8$-Alkyl, Allyl, $C_3$-$C_8$-Cycloalkyl, letzteres unsubstituiert oder mit 1-3 Methylgruppen substituiert, 2-Hydroxyethyl, 3-Hydroxypropyl, 2-Hydroxypropyl
oder
$R^3$ und $R^4$ (zusammen)
= $C_4$-$C_6$-Alkylen, $-(CH_2-CHX)_2Y$mit X = H, $CH_3$;
Y = 0, S
$R^5$, $R^6$ = gleich oder verschieden, H, Alkyl mit 1-4 C-Atomen, verzweigt oder unverzweigt, Cyclohexyl, Phenyl,
n = 4, oder
ein Gemisch von Verbindungen gemäß Formel IV, bei denen $S_n$ einer mittleren statistischen Kettenlänge mit n = 4 entspricht.

Diese Beschleuniger werden entweder allein oder im Verschnitt mit anderen Beschleunigern, z. B. Benzthiazolylsulfenamide, Benzthiazolyldisulfid und 2-Mercapto benzthiazol, und deren Zinksalzen eingesetzt.

Die Verbindungen gemäß den allgemeinen Formeln (I) und (IV) werden nach allgemein bekannten Verfahren in die Kautschukmischungen so eindosiert, daß sie 0,1 bis 5 Teile, bevorzugt 0,1 bis 2 Teile, eines oder mehrerer der s-Triazinsulfenamide und 0,1 bis 10 Teile, bevorzugt 0,1 bis 5 Teile, eines oder mehrerer der N,N′-substitierten Bis-(2,4-diamino-s-triazin-6-yl)tetra oder -polysulfide (Oligosulfide) enthalten, jeweils bezogen auf 100 Teile Kautschuk (phr).

In einer bevorzugten Ausführungsform beläuft sich das molare Verhältnis von s-Triazinsulfenamid zu Tetra-bzw. Polysulfid(en) gemäß Formel (IV) auf 0,3 - 1,5 : 1,0, insbesondere 0,3 - 1,2:1,0, wenn die Kautschukmischung ansonsten keinen freien Schwefel enthält.

Ist freier Schwefel in ihr enthalten, stellt sich das bevorzugte Molverhältnis auf 0,3 - 1,5 (Imid) : 1,0 (Tetra-bzw. Oligosulfid) :0,5 -1,5 (Schwefel), insbesondere 0,3 - 1,2 : 1,0 : 0,5 - 1,5.

Zur Erzielung einer weiteren Variationsbreite der Vulkanisationskinetik kann es sich als zweckmäßig erweisen, die Triazinverbindungen gemäß Formel IV in Form von Gemischen zweier oder mehrerer Individuen einzusetzen, wobei zur Einhaltung der o.a. Anwendungsmengen, insbesondere der bevorzugten molaren Verhältnisse, die Substitution auf äquimolarer Basis vorzunehmen ist.

Auf kinetischen Gründen kann es sich als zweckmäßig erweisen, die N,N′-substituierten Bis-(2,4-diamino-s-triazin-6-yl)-oligosulfide gemäß Formel IV im Gemisch mit konventionellen Beschleunigern der Benzthiazolreihe und/oder Thiuramen einzusetzen, z. B. Benzthiazol bzw. deren Zinksalze und Tetramethylthiuramidisulfid (s. a. J. van Alphen, Rubber Chemicals (1977) Seite 1 - 46).

Mit diesen Dosierungsrichtlinien lassen sich die gestellten Vulkanisationsprobleme lösen, ohne daß es dabei zu einem Vulkanisateigenschaftsverlust kommt.

N,N′-substituierte Bis-(2,4-diamino-s-triazin-6-yl) -oligosulfide gemäß Formel IV sind vorteilhaft zusammen mit Organosilanen einzusetzen, z. B.:
$[(RO)_3Si-(CH_2)_n]_2-S_x$ (V)
oder $(RO)_3-Si-(CH_2)_n-SH$ (VI)
mit x = 2 - 6, bevorzugt 4,
R = $C_1$-$C_6$-Alkyl, Cycloalkyl,
n = 2 oder 3
oder

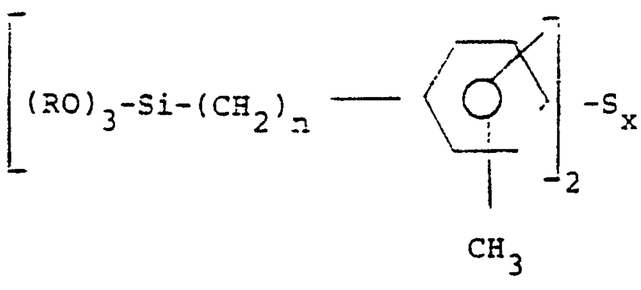

(VII)

mit x = 2 - 6, bevorzugt 3, Alk = Methyl, Ethyl, vorzugsweise Bis-(3-triethoxisilylpropyl)-tetrasulfid (Si 69, Degussa AG) eingesetzt werden, und zwar bei der schwefelfreien Si 69-Vernetzung (DE-PS 22 55 577) und ebenso bei der Herstellung reversionsstabiler Vulkanisate durch Aufbau von equilibrium cure-Systemen gemäß DE-PS 28 48 559.

In allen genannten Fällen fungieren s-Triazinsulfenimide gemäß Formel I als Vulkanisationsverzögerer mit Vernetzungsdichte-erhöhendem Effekt.

Die vulkanisationsverzögernde Wirkung der s-Triazinsulfenimide gemäß Formel I tritt auch ein, wenn die N,N′-substituierten Bis-(2,4-diamino-s-triazin-6-yl) oligosulfide gemäß Formel IV einzeln oder im Gemisch zusammen mit konventionellen Beschleunigern und/oder üblichen Schwefelspendern, wie z. B. Sulfasan(R)R (Morpholindisulfid) eingesetzt werden.

Die Erfindung beinhaltet weiterhin die Anwendung der s-Triazinsulfenimide in vulkanisierbaren Kautschukmischungen als Beschleuniger zusammen mit Schwefel und bzw. oder Schwefelspendern und derartige Kautschukmischungen.

Der Anwendungsbereich der der unter Verwendung von s- Triazinsulfenimiden gemäß Formel I in Kombination mit N,N′-substituierten Bis-(2,4-diamino-s-triazin-6yl) oligosulfiden gemäß Formel IV hergestellten Kautschukmischungen erstrecht sich auf die Herstellung von Reifen, auf technische Artikel, wie z. B. Mischungen für Fördergurte, Keilriemen, Formartikel, Schläuche mit und ohne Einlagen, Walzengummierungen, Auskleidungen, Spritzprofile, Freihandartikel, Folien, Schuhsohlen und Oberteile, Kabel, Vollgummireifen und deren Vulkanisate.

Die gleichen Anwendungsgebiete gelten für Kautschukmischungen, in denen die s-Triazinsulfenimide als alleinige Beschleuniger eingesetzt werden.

Herstellung und Vulkanisation der Kautschukmischungen erfolgen nach den bekannten Verfahren und unter den üblichen Bedingungen.

Die Erfindung wird durch die nachfolgenden Beispiele näher erläutert:

BEISPIEL 1: 2-Diethylamino-4,6-bis-(phthalimidothio)-s-triazin

Man löst 49,2 g (0,2 mol) 2-Diethylamino-4,6-bis-sulfenamoyl-s-triazin und 59,2 g (0,4 mol) Phthalsäureanhydrid in 500 ml Toluol und erhitzt am Wasserabscheider unter Rückfluß zum Sieden. Nach 6 Stunden haben sich 7 ml Wasser abgeschieden. Beim Abkühlen scheiden sich 88,1 g (87% d. Th.) farblose Kristalle vom Schmelzpunkt 257°C ab.

| $C_{23}H_{18}N_6O_4S_2$ (506,56) | | | | |
|---|---|---|---|---|
| Ber. | C 54,53 | H 3,58 | N 16,59 | S 12,66 |
| Gef. | C 54,22 | H 3,65 | N 16,31 | S 13,02 |

BEISPIEL 2: 2-Diethylamino-4,6-bis-(succinimidothio)-s-triazin

In 500 ml Toluol werden 49,2 g (0,2 mol) 2-Diethylamino-4,6-bis-sulfenamoyl-s-triazin, 40,0 g (0,4 mol) Bernsteinsäureanhydrid und 0,2 g p-Toluolsulfonsäure am Wasserabscheider unter Rückfluß zum Sieden erhitzt. Nach 4 Stunden sind 7 ml Wasser abgeschieden. Es werden 400 ml Toluol im Vakuum entfernt. Dabei scheiden sich 51,7 g (63% d. Th.) farblose Kristalle vom Schmelzpunkt 255°C ab.

| $C_{15}H_{18}N_6O_4S_2$ (410,47) | | | | |
|---|---|---|---|---|
| Ber. | C 43,89 | H 4,42 | N 20,47 | S 15,62 |
| Gef. | C 44,00 | H 4,49 | N 20,14 | S 15,56 |

BEISPIEL 3: 2-Diethylamino-4,6-bis-(maleinimido-thio)-s-triazin

In 300 ml Dichlormethan werden 35,0 g (0,142 mol) 2-Diethylamino-4,6-bis-sulfenamoyl-s-triazin, 27,8 g (0,284 mol) Maleinsäureanhydrid und 0,1 g konzentrierte Schwefelsäure am Wasserabscheider unter Rückfluß zum Sieden erhitzt. Nach 12 Stunden haben sich 5 ml Wasser abgeschieden. Nach dem Abkühlen wird die Lösung dreimal mit je 100 ml Wasser neutral gewaschen. Nach dem Trocknen über Natriumsulfat wird das Dichlormethan im Vakuum entfernt und der ölige Rückstand in 300 ml Petrolether gegossen. Dabei scheiden sich 40,8 g (71% d. Th.) farblose Kristalle vom Schmelzpunkt 203°C ab.

| $C_{15}H_{14}N_6O_4S_2$ (406,44) | | | | |
|---|---|---|---|---|
| Ber. | C 44,33 | H 3,47 | N 20,68 | S 15,78 |
| Gef. | C 43,98 | H 3,44 | N 20,49 | S 15,51 |

BEISPIEL 4: 2-Diethylamino-4-ethylamino-6-(phthalimido-thio)-s-triazin

145,2 g (0,6 mol) 2-Diethylamino-4-ethylamino-6-sulfenamoyl-s-triazin und 88,8 g (0,6 mol) Phthalsäureanhydrid werden in 1200 ml Toluol am Wasserabscheider unter Rückfluß zum Sieden erhitzt. Nach 5 Stunden haben sich 10,5 ml Wasser abgeschieden. Beim Abkühlen fallen 173,1 g (78% d. Th.) farblose Kristalle vom Schmelzpunkte 188°C aus.

| $C_{17}H_{20}N_6O_2S$ (372,45) | | | | |
|---|---|---|---|---|
| Ber. | C 54,82 | H 5,41 | N 22,56 | S 8,61 |
| Gef. | C 55,02 | H 5,46 | N 22,42 | S 8,39 |

BEISPIEL 5: 2-Diethylamino-4-ethylamino-6-(succinimido-thio)-s-triazin

188 g (0,78 mol) 2-Diethylamino-4-ethylamino-6-sulfenamoyl-s-triazin, 78 g (0,78 mol) Bernsteinsäureanhydrid und 0,5 g p-Toluolsulfonsäure werden in 1 l Toluol unter Rückfluß zum Siedern erhitzt. Nach 3 Stunden haben sich 14 ml abgeschieden. Die Lösung wird auf 0°C abgekühlt. Dabei fallen 179 g (74% d. Th.) farblose Kristalle vom Schmelzpunkt 170°C aus.

| $C_{13}H_{20}N_6O_2S$ (324,41) | | | | |
|---|---|---|---|---|
| Ber.<br>Gef. | C 48,13<br>C 47,94 | H 6,21<br>H 6,28 | N 25,91<br>N 25,69 | S 9,88<br>S 9,82 |

BEISPIEL 6: 2-Diethylamino-4-ethylamino-6-(1′-2′,3′,6′-tetrahydrophthalimido-thio)-s-triazin

181,5 g (0.75 mol) 2-Diethylamino-4-ethylamino-6-sulfenamoyl-s-triazin,114,0 g (0,75 mol) 1,2,3,6-Tetrahydrophthalsäureanhydrid und 0,5 g konzentrierte Phosphorsäure werden in 1000 ml Chloroform am Wasserabscheider unter Rückfluß zum Sieden erhitzt. Nach 5 Stunden sind 13 ml Wasser abgeschieden. Nach dem Abkühlen wird die Lösung dreimal mit je 300 ml Wasser neutral gewaschen und die organische Phase über Natriumsulfat getrocknet. Es werden 700 ml Chloroform in Vakuum abdestilliert. Beim Abkühlen fallen 152 g (54% d. Th.) farblose Kristalle vom Schmelzpunkt 138° aus.

| $C_{17}H_{24}N_6O_2S$ (376,48) | | | | |
|---|---|---|---|---|
| Ber.<br>Gef. | C 54,24<br>C 54,50 | H 6,43<br>H 6,29 | N 22,32<br>N 22,09 | S 8,52<br>S 8,44 |

BEISPIEL 7: 2-Diethylamino-4-ethylamino-6-(3′,4′,5′-6′-tetrachlorophthalimido-thio)-s-triazin

In 250 ml Toluol werden 12,1 g (0,05 mol) 2-Diethylamino-4-ethylamino-6-sulfenamoyl-s-triazin und 14,3 g (0,05 mol) 3,4,5,6-Tetrachlorophthalsäureanhydrid am Wasserabscheider unter Rückfluß zum Sieden erhitzt. Nach 2 Stunden haben sich ca. 0,9 ml Wasser abgeschieden. Die Lösung wird auf 0°C abgekühlt. Dabei fallen 19,0 g (75% d. Th.) farblose Kristalle vom Schmelzpunkt 270°C aus.

| $C_{17}H_{16}Cl_4N_6O_2S$ (510,23) | | | | | |
|---|---|---|---|---|---|
| Ber.<br>Gef. | C 40,02<br>C 40,41 | H 3,16<br>H 3,24 | Cl 27,79<br>Cl 27,34 | N 16,47<br>N 16,30 | S 6,28<br>S 5,97 |

BEISPIEL 8: 2-Diethylamino-4-ethylamino-6-(dichlormaleinimido-thio)-s-triazin

12,1 g (0,05 mol) 2-Diethylamino-4-ethylamino-6-sulfenamoyls-triazin und 8,35 g (0,05 mol) Dichlormaleinsäureanhydrid werden in 150 ml Dichlormethan am Wasserabscheider unter Rückfluß zum Sieden erhitzt. Innerhalb einer Stunde scheiden sich ca. 0,8 ml Wasser ab. Beim Abkühlen fallen 9,4 g (48% d. Th.) hellgelbe Kristalle vom Schmelzpunkt 126°C aus.

| $C_{13}H_{16}Cl_2N_6O_2S$ (391,86) | | | | | |
|---|---|---|---|---|---|
| Ber.<br>Gef. | C 39,85<br>C 39,70 | H 4,12<br>H 4,30 | Cl 18,09<br>Cl 18,25 | N 21,45<br>N 21,52 | S 8,18<br>S 7,90 |

Prüfungsnormen

Die physikalischen Prüfungen wurden bei Raumtemperatur nach folgenden Normvorschriften ausgeführt:

| | | gemessen in |
|---|---|---|
| Spannungswert 300% | DIN 53 504 | MPa |
| Inkubationszeit $t_I$ | DIN 53 529 | min |
| Scorchzeit | ASTM D 2084 | min |
| Dmax-Dmin | DIN 53 529 | Nm |
| $t_{80}$ - $t_{20\%}$ | DIN 53 529 | min |
| $t_{90}$ - $t_{10\%}$ | DIN 53 529 | min |
| $t_{10\%}$ | DIN 53 529 | min |

In den Anwendungsbeispielen werden folgende Namen und Abkürzungen benutzt, deren Bedeutung im folgenden angegeben wird:
SBR 1500 : Styrol-Butadien-Kautschuk (Synthesekautschuk)
RSS : Ribbed Smoked Sheet (Naturkautschuk)
CORAX(R) N 220 : Ruß, Oberfläche (BET) 120 $m^2/g$ (Degussa)
Naftolen(R) ZD : Weichmacher aus Kohlenwasserstoffen
Vulkanox(R) 4010NA: N-Isopropyl-N′-phenyl-p-phenylen-diamin
Vulkanox(R) HS : Poly-2,2,4-trimethyl-1,2-dihydrochinolin
Protektor(R) G 35 : Ozonschutzwachs
Vulkacit(R) MOZ : Benzthiazolyl-2-morpholino-sulfenamid
Vulkalent(R) E : N-Phenyl-N-(trichlormethyl-sulfenyl)benzolsulfonamid
Ultrasil(R) VN 3 : gefällte Kieselsäure (Degussa)
Gran. : Granulat
VZ a : 2-Diethylamino-4,6-bis-(phthalimido-thio)-s-triazin
VZ b : 2-Diethylamino-4,6-bis-(succinimido-thio)-s-triazin
VZ c : 2-Diethylamino-4-ethylamino-6-(phthalimido-thio)-s-triazin
VZ d : 2-Diethylamino-4-ethylamino-6-(succinimido-thio)-s-triazin
VZ e : 2-Diethylamino-4-ethylamino-6-(1′,2′,3′,6′-tetrahydrophthalimido-thio)-s-triazin

BEISPIEL 9: Vergleich von s-Triazinsulfenimiden mit Trichlormethylthiohydantoin in N 220-gefülltem NR mit N,N'-substituiertem Bis-(2-ethylamino-4-diethylamino-s-triazin-6-yl)-tetrasulfid (V 480) als Beschleuniger

| | 1 | 2 | 3 | 4 |
|---|---|---|---|---|
| RSS 1, ML (1+4)=70-80 | 100 | 100 | 100 | 100 |
| CORAX N 220 | 50 | 50 | 50 | 50 |
| ZnO RS | 5 | 5 | 5 | 5 |
| Stearinsäure | 2 | 2 | 2 | 2 |
| Naftolen ZD | 3 | 3 | 3 | 3 |
| Protektor G 35 | 1 | 1 | 1 | 1 |
| Vulkanox 4010 NA | 2,5 | 2,5 | 2,5 | 2,5 |
| Vulkanox BS | 1,5 | 1,5 | 1,5 | 1,5 |
| V 480 | 1,5 | 1,5 | 1,5 | 1,5 |
| Trichlormethylthiohydantoin | - | 0,8 | - | - |
| VZ a | - | - | 1,3 | - |
| VZ b | - | - | - | 1,08 |
| Schwefel | 0,8 | 0,8 | 0,8 | 0,8 |
| $t_{10\%}$ (160°C), min. | 3,8 | 5,8 | 6,8 | 6,8 |
| $\frac{Dmax-D(max + 60')}{Dmax - Dmin}$, % | 2,9 | 5,7 | 2,8 | 2,7 |
| Mooney-Scorch t5 (130°C), min. | 8,3 | 18,5 | 22,4 | 21,5 |
| **Vulkanisatdaten bei 160°C** | | | | |
| Spannungswert 300% | 10,8 | 11,8 | 12,2 | 12,5 |

Wie Beispiel 9 zeigt, führt der Zusatz der beanspruchten Substanzen zu N,N'-substituiertem Bis-(2-ethylamino-4-diethylamino-s-triazin-6-yl)-tetrasulfid zu einer deutlichen Verlängerung der Scorchzeit bei Verarbeitungs- und Vulkanisationstemperatur und gleichzeitig zu einer Modulerhöhung. Diese Effekte sind dabei gegenüber der äquimolaren Menge Trichlormethylthiohydantoin stärker ausgeprägt.

Gleichzeitig läßt sich feststellen, daß die beanspruchten Substanzen die durch Trichlormethylthiohydantoin hervorgerufene Reversionsneigung nicht besitzen.

| BEISPIEL 10: Wirkung von s-Triazinsulfenimiden in Bis-(2-ethylamino-4-diethylamino-s-triazin-6-yl)-tetrasulfid (V 480) -haltigem, Ruß N 220/Kieselsäure-gefülltem NR | | | |
|---|---|---|---|
| | 5 | 6 | 7 |
| RSS 1, ML (1+4)=70-80 | 100 | 100 | 100 |
| CORAX N 220 | 25 | 25 | 25 |
| Ultrasil VN 3 Gran. | 25 | 25 | 25 |
| ZnO RS | 5 | 5 | 5 |
| Stearinsäure | 2 | 2 | 2 |
| Naftolen ZD | 3 | 3 | 3 |
| Protektor G 35 | 1 | 1 | 1 |
| Vulkanox 4010 NA | 2,5 | 2,5 | 2,5 |
| Vulkanox HS | 1,5 | 1,5 | 1,5 |
| Vulkacit MOZ | 1,43 | - | - |
| V 480 | - | 1,5 | 1,5 |
| VZ d | - | - | 0,86 |
| Schwefel | 1,5 | 0,8 | 0,8 |
| $t_{10\%}$ (160°C), min. | 5,9 | 5,1 | 6,3 |

Beispiel 10 zeigt, daß auch bei einer Füllstoffmischung aus Ruß und Kieselsäure die geringere Anvulkanisationszeit des Bis-(2-ethylamino-4-diethylamino-s-triazin-6-yl)-tetrasulfid-Beschleunigers (Mischung 6) auf den Bereich der Sulfenamid-Beschleunigung (Mischung 5) angehoben werden kann.

BEISPIEL 11: Vergleich von s-Triazinsulfenimiden mit Vulkalent E in N 220-gefülltem SBR mit Triazintetrasulfid-Beschleunigern

| | 8 | 9 | 10 |
|---|---|---|---|
| SBR 1500 | 100 | 100 | 100 |
| CORAX N 220 | 50 | 50 | 50 |
| ZnO RS | 5 | 5 | 5 |
| Stearinsäure | 2 | 2 | 2 |
| Naftolen ZD | 3 | 3 | 3 |
| Vulkanox 4010 NA | 2,5 | 2,5 | 2,5 |
| Vulkanox HS | 1,5 | 1,5 | 1,5 |
| Protektor G 35 | 1 | 1 | 1 |
| V 480 | 1,5 | 1,5 | 1,5 |
| Vulkalent E | - | 0,6 | - |
| VZ a | - | - | 0,65 |
| Schwefel | 1,8 | 1,8 | 1,8 |
| Dmax-Dmin (160°C), Nm | 11,20 | 11,45 | 12,57 |
| $t_{10\%}$ (160°C), min. | 5,2 | 5,6 | 7,3 |
| $\frac{Dmax-D(max + 60')}{Dmax - Dmin}$, % | 7,0 | 6,3 | 3,1 |

Der äquimolare Ersatz des handelsüblichen Verzögerers Vulkalent E (Mischung 9) durch ein s-Triazinsulfenimid (Mischung 10) in rußgefülltem SBR zeigt die deutlich höhere scorchverlängernde Wirkung der beanspruchten Substanzen und die größere Wirksamkeit bezüglich der Vernetzung.

Zusätzlich weist Beispiel 11 nach, daß die beanspruchten Substanzen in synthetischen Kautschuktypen in Verbindungen mit Triazintetrasulfid-Beschleunigern eine reversionssenkende Wirkung besitzen.

| BEISPIEL 12: Nachweis der Wirksamkeit von s-Triazinsulfenimiden in Ruß N 220/Kieselsäure-gefülltem SBR | | |
|---|---|---|
| | 11 | 12 |
| SBR 1500 | 100 | 100 |
| CORAX N 220 | 25 | 25 |
| Ultrasil VN 3 Gran. | 25 | 25 |
| ZnO RS | 5 | 5 |
| Stearinsäure | 2 | 2 |
| Naftolen ZD | 3 | 3 |
| Vulkanox 4010 NA | 2,5 | 2,5 |
| Vulkanox HS | 1,5 | 1,5 |
| Protektor G 35 | 1 | 1 |
| V 480 | 1 | 1 |
| VZ a | - | 0,55 |
| Schwefel | 1,8 | 1,8 |
| Dmax-Dmin (160°C), Nm | 6,21 | 7,82 |
| $t_{10\%}$ (160°C), min. | 7,3 | 8,8 |
| $t_{90}$ - $t_{10\%}$, min. | 19,3 | 15,6 |

Beispiel 12 zeigt, daß s-Triazinsulfenimide in Verbindung mit Triazintetrasulfid-Beschleunigern in synthetischen Kautschuken einen Beitrag zur Vernetzungsausbeute liefern, die Scorchzeit verlängern und zusätzlich die Vernetzungsgeschwindigkeit erhöhen.

| BEISPIEL 13: Vergleich von s-Triazinsulfenimiden mit V 480 als Beschleuniger in N 220-gefülltem NR | | | |
|---|---|---|---|
| | 13 | 14 | 15 |
| RSS 1, ML (1+4)=70-80 | 100 | 100 | 100 |
| CORAX N 220 | 50 | 50 | 50 |
| ZnO RS | 5 | 5 | 5 |
| Stearinsäure | 2 | 2 | 2 |
| Naftolen ZD | 3 | 3 | 3 |
| Protektor G 35 | 1 | 1 | 1 |
| Vulkanox 4010 NA | 2,5 | 2,5 | 2,5 |
| Vulkanox HS | 1,5 | 1,5 | 1,5 |
| V 480 | 1,5 | - | - |
| VZ d | - | 1,5 | - |
| VZ e | - | - | 1,5 |
| Schwefel | 0,8 | 1,02 | 1,02 |
| Dmax-Dmin (160°C), Nm | 8,60 | 8,51 | 7,80 |
| $t_{10\%}$ (160°C), min. | 3,9 | 9,4 | 9,0 |
| $t_{80}$-$t_{20\%}$ (160°C), min | 3,0 | 2,8 | 2,9 |
| Vulkanisatdaten bei 160° C Spannungswert 300% | 10,6 | 10,3 | 9,3 |

In Beispiel 13 wird die beschleunigende Wirkung der s-Triazinsulfenimide (Mischung 14 und 15) im Vergleich zur Triazintetrasulfid-Beschleunigung (Mischung 13) für Naturkautschuk nachgewiesen. Dabei ist bei gleicher Vernetzungsgeschwindigkeit eine deutlich höhere Scorchsicherheit feststellbar.

BEISPIEL 14: Vergleich von s-Triazinsulfenimiden mit V 480 als Beschleuniger in N 220-gefülltem SBR

| | 16 | 17 | 18 |
|---|---|---|---|
| Buna 1500 | 100 | 100 | 100 |
| CORAX N 220 | 50 | 50 | 50 |
| ZnO RS | 5 | 5 | 5 |
| Stearinsäure | 2 | 2 | 2 |
| Naftolen ZD | 3 | 3 | 3 |
| Vulkanox 4010 NA | 2,5 | 2,5 | 2,5 |
| Vulkanox HS | 1,5 | 1,5 | 1,5 |
| Protektor G 35 | 1 | 1 | 1 |
| V 480 | 1,5 | - | - |
| VZ d | - | 1,5 | - |
| VZ e | - | - | 1,1 |
| Schwefel | 1,8 | 2,0 | 2,1 |
| Dmax-Dmin (160°C), Nm | 10,61 | 10,25 | 7,58 |
| $t_{10\%}$ (160°C), min. | 5,3 | 11,8 | 10,2 |
| $\frac{Dmax-D(max + 60')}{Dmax - Dmin}$, % | 8,1 | 6,5 | 0 |
| **Vulkanisatdaten bei 160°C** | | | |
| Spannungswert 300% | 11,6 | 11,1 | 8,5 |

Beispiel 14 weist die beschleunigende Wirkung der s-Triazinsulfenimide (Mischung 17, 18) im Vergleich zur Triazintetrasulfid-Beschleunigung (Mischung 16) für Synthesekautschuk nach.

Neben deutlich höherer Scorchsicherheit ist - wie beim Einsatz als Verzögerer in Synthesekautschuk - ein positiver Effekt auf die Reversion festzustellen.

## Ansprüche

1. Verfahren zur Herstellung von s-Triazinsulfenimiden der allgemeinen Formel

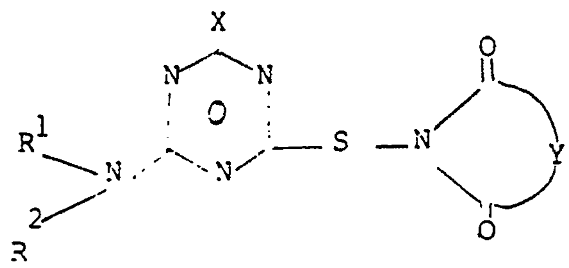 (I)

,

in der bedeuten:

X:

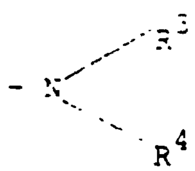

oder

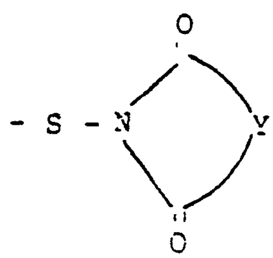

$R^1$, $R^2$, $R^3$ und $R^4$, gleich oder verschieden:

Wasserstoff, Alkyl mit 1 bis 4 Kohlenstoffatomen, Alkenyl mit 3 bis 5 Kohlenstoffatomen, Cycloalkyl mit 3 bis 8 Kohlenstoffatomen.

$R^1$ mit $R^2$ und/oder $R^3$ mit $R^4$ können ringförmig miteinander verknüpft sein, so gestalt,

daß 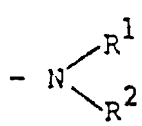 und/oder 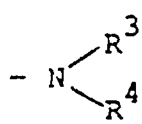 gemeinsam

einen Pyrrolidino-, Piperidino-, Morpholino-, 3,5-Dimethylmorpholino- oder 4-N-Alkylpiperazinoring bilden;

Y : die Gruppierungen

$- CH_2 - CH_2 -$

$- CH_2 - CH_2 - CH_2 -$

$- CH(CH_3) - CH_2 -$

$- CH = CH -$

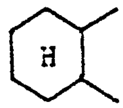

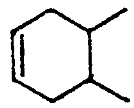

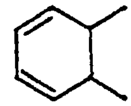

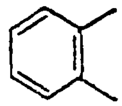

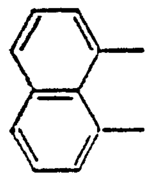

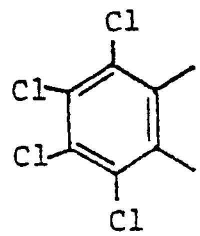

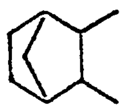

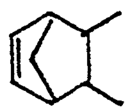

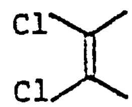 ,

dadurch gekennzeichnet, daß man ein s-Triazinsulfenamid der Formel

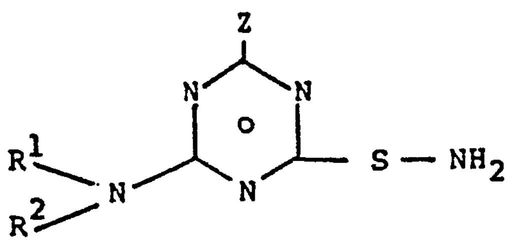 ( II ) ,

in der bedeuten

Z:

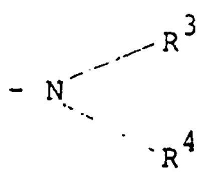

oder

- S - $NH_2$

und $R^1$, $R^2$, $R^3$ und $R^4$ die oben aufgeführte Bedeutung haben, mit einem cyclischen Dicarbonsäureanhydrid der Formel

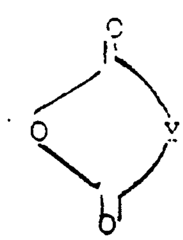 (III),

in der Y die oben aufgeführte Bedeutung besitzt, in einem inerten, wasserunlöslichen, organischen Lösungsmittel bei Temperaturen zwischen 20 °C und 150 °C, vorzugsweise der Siedetemperatur des

verwendeten Lösungsmittel, umsetzt.

2. Verfahren gemäß Anspruch 1, dadurch gekennzeichnet, daß man das sich während der Reaktion bildende Wasser gleichzeitig azeotrop abdestilliert.

3. Verfahren gemäß Anspruch 2, dadurch gekennzeichnet, daß man als wasserschleppendes Lösungsmittel chlorierte, aliphatische oder aromatische Kohlenwasserstoffe verwendet.

4. Verfahren gemäß den Ansprüchen 1 bis 3, dadurch gekennzeichnet, daß man Schwefelsäure, Phosphorsäure, p-Toluolsulfonsäure oder einen sauren Ionenaustauscher als Katalysator einsetzt.

5. Verwendung von gemäß einem oder mehreren der Ansprüche 1 bis 4 hergestellten Verbindungen der allgemeinen Formel I in Kombination mit oligosulfidischen Verbindungen der allgemeinen Formel

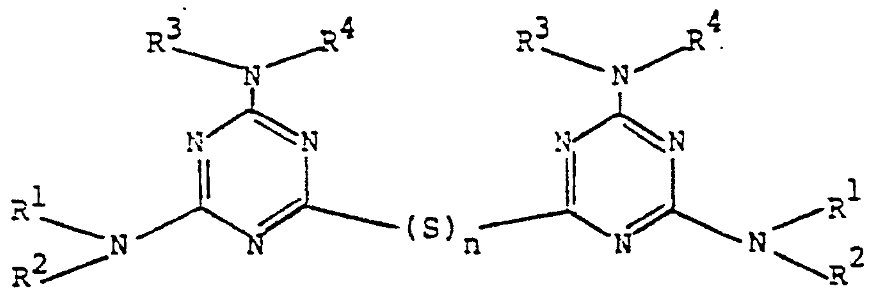

IV

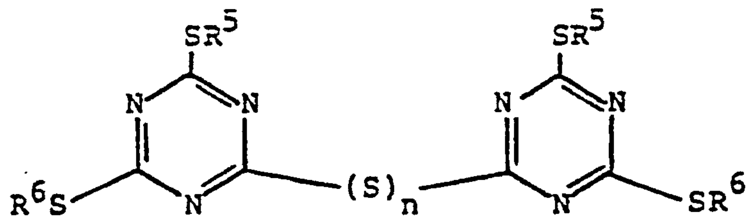

in welcher bedeuten:

$R^1$, $R^2$ = H;

$R^2$, $R^3$, $R^4$ = H, $C_1$-$C_8$-Alkyl, Allyl, $C_3$-$C_8$-Cycloalkyl,

letzteres unsubstituiert oder mit 1-3 Methylgruppen substituiert, 2-Hydroxyethyl, 3-Hydroxypropyl, 2-Hydroxypropyl

oder

$R^3$ und$R^4$ (zusammen)

= $C_4$-$C_6$-Alkylen, $-(CH_2-CHX)_2Y$mit X = H, $CH_3$; Y = O,S

$R^5$, $R^6$ = gleich oder verschieden, H, Alkyl mit 1-4 C-Atomen, verzweigt oder unverzweigt, Cyclohexyl, Phenyl,

n = 4, oder

ein Gemisch von Verbindungen gemäß Formel IV, bei denen $S_n$einer mittleren statistischen Kettenlänge mit n = 4 entspricht,

in mit Schwefel vulkanisierbaren Kautschukmischungen als Vulkanisationsverzögerer mit einem die Vernetzungsdichte erhöhendem Effekt.

6. Vulkanisierbare Kautschukmischungen gemäß Anspruch 5, dadurch gekennzeichnet, daß sie 0,1 bis 5 Teile s-Triazinsulfenimide gemäß Formel I enthalten, sowie 0,1 bis 10 Teile N,N′-substituierter Bis-(2,4-diamino-s-triazin-6-yl)-oligosulfide gemäß Formel IV, sowie 0,1 bis 10 Teile Schwefel, jeweils bezogen auf 100 Teile Kautschuk, wobei die drei Komponenten im molaren Verhältnis von 0,3-1,5:1 :0,5 - 1,5, vorzugsweise 0,3-1,2 : 1 : 0,5-1,5 , stehen.

7. Vulkanisierbare Kautschukmischungen gemäß Anspruch 5, dadurch gekennzeichnet, daß sie 0,1 bis 5 Teile s-Triazinsulfenimide gemäß Formel I und 0,1 bis 10 Teile N,N′-substituierter Bis-(2,4-diamino-s-triazin-6-yl)-oligosulfide gemäß Formel IV enthalten, jeweils bezogen auf 100 Teile Kautschuk, wobei die beiden Komponenten im molaren Verhältnis von 0,3-1,5 : 1 stehen, und die Mischung keinen freien Schwefel enthält.